# EUROPÄISCHE PATENTSCHRIFT

(11) **EP 0 585 552 B1**
(45) Veröffentlichungstag und Bekanntmachung des Hinweises auf die Patenterteilung: **29.01.2003**
(21) Anmeldenummer: 93109742.2
(22) Anmeldetag: 18.06.1993
(51) Int. Cl.: C07K 14/00, A61K 38/00, A61K 38/46

(54) **Verwendung von Komplement-Inhibitoren zur Herstellung eines Arzneimittels zur Prophylaxe und Therapie von entzündlichen Darm- und Hauterkrankungen sowie Purpura**
Use of complement inhibitors for preparing a medicament for the prophylaxis and treatment of inflammatory intestinal and skin diseases as well as purpura
Emploi des inhibiteurs du complément pour la préparation de médicaments pour la prophylaxe et le traitement des maladies inflammatoires intestinales et de la peau et de la Purpura

(30) Priorität: 09.07.1992 DE 4222534
(43) Veröffentlichungstag der Anmeldung: 09.03.1994
(73) Patentinhaber: Centeon Pharma GmbH, 35041 Marburg (DE)
(72) Erfinder: Römisch, Jürgen, D-3550 Marburg (DE); Paques, Eric-Paul, D-3550 Marburg (DE); Bartlett, Robert, D-6100 Darmstadt-Arheiligen (DE); Dickneite, Gerhard, D-3550 Marburg (DE)

(56) Entgegenhaltungen:
- EP-A- 0 222 611
- WO-A-92/10205
- DE-A- 2 617 202
- US-A- 5 135 916

## Beschreibung

Die Erfindung betrifft die Verwendung von Komplement-Inhibitoren, besonders von C1-Inaktivator oder der Faktoren I oder H, zur Herstellung eines Arzneimittels zur Prophylaxe und Therapie von chronisch entzündlichen Darmerkrankungen, inflammatorischen Hautkrankheiten und Purpura.

Das Komplement-System besteht aus einer Reihe von Pro teinen, in der Mehrzahl Proteasen, die nach initialer Aktivierung zur Bildung des terminalen Lysekomplexes und somit zur Zerstörung von Zielzellen führen. Ausgelöst werden solche Prozesse "klassisch" durch Bildung von Immunkomplexen bzw. Kontaktaktivierung und "alternativ" durch körperfremde Strukturen, wie Bakterien und deren Lipopolysaccharide. Beide Aktivierungswege münden in die Erzeugung der Komponente C3b, die zusammen mit C5-C9 den Membran-Angriffskomplex initiiert. Proteolytische Aktivierung der Komponenten C3, C4 und C5 führt zur Freisetzung der Anaphylatoxine C3a, C4a und C5a, die chemotaktisch auf Entzündungszellen wirken.

Die wichtigsten physiologischen Regulatoren des Komplementsystems sind die inhibitorisch wirksamen Proteine C1-Inaktivator, Faktor I (auch als C3b-Inaktivator bezeichnet) und dessen Akzelerator Faktor H. Der erstgenannte Inhibitor entfaltet seine Wirkung an der Initiationsstelle des "klassischen" Weges durch Interaktion mit der aktivierenden Protease. Dagegen ist der Faktor I eine Protease, deren katalytische Wirkung durch Faktor H erheblich gesteigert wird, die das C3b-Molekül und auch das C4b-Molekül durch partielle Dagradation inaktiviert und somit an der Mündung von "klassischen" und "alternativen" Weg regulatorisch/inhibierend eingreift.

Die Aktivierung des Komplement-Systems wurde im Verlauf einer Reihe von Autoimmun-Erkrankungen beobachtet, darunter Lupus erythematodes und rheumatoide Arthritis. Diese Aktivierungsprozesse sind allgemein mit einem Verbrauch der beteiligten Faktoren, insbesondere der Inhibitoren, verbunden. Obwohl oft auch eine Erhöhung der Komplementfaktoren im Verlauf der Akut-Phase-Reaktion zu beobachten ist, reicht die inhibitorische Kapazität nicht aus, eine Komplementaktivierung und die daraus resultierenden Folgen zu kontrollieren. Eine Substitution mit diesen Regulatoren bzw. prophylaktische Verabreichung erscheint daher sinnvoll. Die therapeutische Wertigkeit der Faktoren I und H bei der Glomerulonephritis wurde beschrieben in EP-A-0 222611.

Die Äthiopathogenese chronisch entzündlicher Darmerkrankungen, vor allem "Morbus Crohn" und "Colitis ulcerosa", ist bis heute nicht geklärt. Ausgelöst durch einen "Primärstimulus" wird ein Immunprozeß in Gang gesetzt, dem die Gewebe-Infiltration von Entzündungszellen und schließlich eine Zellund Gewebeschädigung folgen. Klinische Symptome sind Ulzera; Fistelbildungen und häufige blutige Stühle/Diarrhoe, die mit Fieberschüben und krampfartigen Schmerzen einhergehen.

Bisher konnten keine für diese Erkrankungen pathognomischen Autoantikörper oder autoreaktive T-Zellen definiert werden, die solche entzündlichen Prozesse als Autoimmunkrankheit ausweisen. Eine Beteiligung von Immunprozessen wird jedoch nicht ausgeschlossen, die eine Komplementaktivierung zur Folge haben können. Dabei können die entstehenden Anaphylatoxine C3a, C4a, C5a zur Anlockung von Entzündungszellen beitragen. Auffällig sind auch die in der frühen Krankheitphase auftretenden Ödeme der Darmschleimhaut, wie sie typischerweise nach Aktivierung des Komplementsystems beobachtet werden.

Auch in Hautkrankheiten, wie z.B. bei pustulären Dermatosen, Dermatitiden, oder Psoriasis, werden erhöhte C5a-Spiegel gemessen, die eindeutig die Aktivierung des Komplementsystems belegen und die Anlockung von Entzündungszellen vermitteln. Diese wiederum tragen maßgeblich zur Ausprägung des Krankheitsbildes bei.

Auch beim Krankheitsbild der Purpura können z.B. endotheliale Zellen ödematosen Veränderungen unterworfen sein, die zu kapillären Dilatation führen. Klinisch manifestiert sich dieser Zustand als Erythem/Ödem z.B. an der Stelle der Hautläsion. Generell wird unter dem Begriff "Purpura" die Extravasation von "geformten Blutelementen" aus dermalen Blutgefäßen in die Haut (z.B. Dermis) verstanden. Bei der idiopathischen thrombozytopenischen Purpura tritt typischerweise die Blutungsneigung mit Hämatombildung (Extravasation) auf, die durch eine Verarmung an Blutplättchen maßgeblich unterstützt wird. Eine "entzündliche" Purpura geht häufig mit einer Vasculitis einher, die durch Immunkomplexe bedingt sein kann.

Demnach haben chronisch entzündliche Darmerkrankungen sowie Purpura (verbunden mit Hautnekrosen) und entzündliche Hautkrankheiten die Extravasation von Flüssigkeit (aus Blutgefäßen) gemeinsam. Die häufig resultierende Ödembildung, wie auch die Anlockung und Infiltration von Entzündungszellen in das inflammatorische Gewebe sind Vorgänge, wie sie häufig nach Aktivierung des Komplementsystems beobachtet werden.

Zur Untersuchung der beschriebenen Vorgänge und der pharmakologischen Prüfung von therapeutisch wirksamen Substanzen wird oft das als "Arthus-Reaktion" bekannte Tiermodell herangezogen (siehe Beispiel 1).

Wir fanden nun, daß die Komplement-Inhibitoren C1-Inaktivator und die Faktoren I und H eine hemmende Wirkung auf die Arthus-Reaktion ausüben.

Für die Prophylaxe und Therapie entzündlicher Hautkrankheiten, wie z.B. pustuläre Dermatosen, Dermatitiden oder Psoriasis und Darmerkrankungen, besonders Morbus Crohn und Colitis ulcerosa sowie entzündlicher Purpura (verbunden mit Hautnekrosen) können demnach Komplement-Inhibitoren, besonders C1-Inaktivator und/oder Faktor I und/oder Faktor H-haltige Lösungen verwendet werden.

Gegenstand der Erfindung ist die Verwendung von C1-Inaktivator, Faktor I und/oder Faktor H zur Herstellung eines Arzneimittels für die Therapie oder Prophylaxe von akuten, nekrotisierenden, inflammatorischen Läsionen von Blutgefäßen.

Besonders geeignet sind der C1-Inaktivator und die Faktoren I und H oder Kombinationen dieser.

Bevorzugt werden gereinigte Inhibitoren verwendet, die auf eine dem Fachmann bekannte Weise aus Blutplasma präpariert werden können.

Auch auf gentechnischem Wege exprimierte und gereinigte Inhibitoren können dafür eingesetzt werden.

Bei der Applikation anf intravenöse (Bolus oder Infusion), intramuskuläre oder subkutane Weise werden folgende Dosierungen verwendet:
- C1-Inaktivator :: 1-5000 IU/kg Körpergewicht (KG)
pro Tag, bevorzugt 5-500 IU/kg x Tag.
- Faktor I:: 0.005-100 mg/kg KG pro Tag,
bevorzugt 0.01-50 mg/kg x Tag.
- Faktor H:: 0.005-100 mg/kg KG pro Tag,
bevorzugt 0.01-50 mg/kg x Tag.

Die Inhibitoren können separat oder als Kombination angewendet werden. Allgemein ist die Applikation nur eines dieser Inhibitoren ausreichend.

Eine Kombination der hemmenden Proteine erscheint besonders dann sinnvoll, wenn die Aktivierung beider Komplementwege, des klassischen und des alternativen, nicht auszuschließen ist. Cl-Inaktivator allein inhibiert zwar die Kontaktaktivierung des klassischen Weges und dadurch auch die darauf folgende Freisetzung der Anaphylatoxine, hat jedoch keinen Einfluß auf den alternativen Weg. Dagegen kann durch die Verwendung der Faktoren I und/oder H die Erzeugung des terminalen Lysekomplexes kontrolliert werden. Der Verbrauch der Komplementfaktoren bis zur Mündungsstelle beider Aktivierungswege ist dagegen durch alleinige Verwendung der Faktoren I und H nicht aufzuhalten.

### Beispiel:

Die Arthus-Reaktion, als akute, nekrotisierende, inflammatorische Läsion von Blutgefäßen bezeichnet, wird durch Immunisierung und anschließende Provokation mit einem körperfremden Antigen oder direkt mit einem gegen die Testspezies gerichteten Antikörper ausgelöst. Extravasation, Ödembildung und Anlockung von Entzündungszellen kennzeichnen die Arthus-Reaktion. Als Meßparameter dient die Ausprägung der Ödembildung einer Ratten-Hinterpfote. Je besser diese Ödembildung vermindert/verhindert werden kann, desto wirkungsvoller ist die als Therapeutikum getestete Substanz.

Die Ergebnisse der Untersuchung der Komplement-Inhibitoren auf die Arthus-Reaktion sind in Tabelle 1 aufgeführt. Sowohl C1-Inaktivator und Faktor H als auch Faktor I haben nach i.v. Bolusapplikation einen hemmenden Einfluß auf die Ödembildung. Auch subkutan appliziert verhindert Faktor I signifikant die Schwellung.

**Tabelle 1:**

| Wirkung von C1-Inaktivator, Faktor I und Faktor H auf die Arthusreaktion in der Rattenpfote. | | | |
|---|---|---|---|
| Die Inhibition (%) der Schwellung wurde im Vergleich zu einer Kontrollgruppe (Placebo) ermittelt. Als Positivkontrolle wurde eine Gruppe mit Prednisolon behandelt. Die Testsubstanzen wurden eine Stunde vor Provokation appliziert (i.v. = intravenös; s.c. = subkutan; p.o. = per os), die Pfotenschwellung vier Stunden danach gemessen (10 Tiere/Gruppe). | | | |

| **Substanz** | **Dosis** | **Applikation** | **Inhibition (%)** |
|---|---|---|---|
| | | | |
| C1-Inaktivator | 100 IU/kg | i.v. | 12 |
| | 200 IU/kg | i.v. | 38 |
| | | | |
| Faktor I | 0.05 mg/kg | i.v. | 43 |
| | 0.50 mg/kg | i.v. | 55 |
| | 5.00 mg/kg | i.v. | 65 |
| | | | |
| | 10.00 mg/kg | s.c. | 59 |
| | 20.00 mg/kg | s.c. | 57 |
| | | | |
| Faktor H | 1.0 mg/kg | i.v. | 35 |
| | 10.0 mg/kg | i.v. | 44 |
| | | | |
| Prednisolon | 29.0 mg/kg | p.o. | 70 |

## Patentansprüche

1. Verwendung von C1-Inaktivator, Faktor I und/oder Faktor H zur Herstellung eines Arzneimittel für die Therapie oder Prophylaxe von akuten, nekrotisierenden, inflammatorischen Läsionen von Blutgefäßen.

2. Verwendung nach Anspruch 1, wobei die akuten, nekrotisierenden, inflammatorischen Läsionen von entzündlichen Haut- und Darmerkrankungen oder Purpura herrühren.

3. Verwendung nach mindestens einem der Ansprüche 1 bis 2, **dadurch gekennzeichnet, dass** das Arzneimittel 1 bis 5000 IU/kg x Tag C1-Inaktivator oder 0,005 bis 100mg/kg x Tag Faktor I oder 0,005 bis 100 mg/kg x Tag Faktor H einzeln, oder in Kombination, enthält.

4. Verwendung nach Anspruch 3, **dadurch gekennzeichnet, dass** das Arzneimittel 5 bis 500 IU/kg x Tag C1-Inaktivator oder 0,01 bis 50 mg/kg x Tag Faktor I oder 0,01 bis 50 mg/kg x Tag Faktor H einzeln, oder in Kombination, enthält.

## Claims

1. The use of C1 inactivator, factor I and/or factor H for the preparation of a pharmaceutical for the therapy or prophylaxis of acute, necrotizing, inflammatory lesions of blood vessels.

2. The use as claimed in claim 1, where the acute, necrotizing, inflammatory lesions are caused by inflammatory skin disorders and intestinal disorders or purpura.

3. The use as claimed in at least one of claims 1 and 2, wherein the pharmaceutical contains 1-5000 IU/kg × day C1 inactivator or 0.005-100 mg/kg × day factor I or 0.005-100 mg/kg × day factor H singly or in combination.

4. The use as claimed in claim 3, wherein the pharmaceutical contains 5-500 IU/kg × day C1 inactivator or 0.01-50 mg/kg × day factor I or 0.01-50 mg/kg × day factor H, singly or in combination.

## Revendications

1. Utilisation d'inactivateur C1, facteur I et/ou facteur H pour la fabrication d'un médicament pour la prévention et le traitement de lésions inflammatoires nécrosantes aiguës de vaisseaux sanguins.

2. Utilisation selon la revendication 1, dans laquelle les lésions inflammatoires nécrosantes aiguës proviennent de maladies inflammatoires de la peau et de l'intestin ou de purpura.

3. Utilisation selon au moins une des revendications 1 et 2, **caractérisée en ce que** le médicament contient de 1 à 5 000 UI/kg × jour d'inactivateur C1 ou de 0,005 à 100 mg/kg × jour de facteur I ou de 0,005 à 100 mg/kg × jour de facteur H, seuls ou en association.

4. Utilisation selon la revendication 3, **caractérisée en ce que** le médicament contient de 5 à 500 UI/kg × jour d'inactivateur C1 ou de 0,01 à 50 mg/kg × jour de facteur I ou de 0,01 à 50 mg/kg × jour de facteur H, seuls ou en association.
